# EUROPEAN PATENT APPLICATION

(11) **EP 1 243 927 A1**
(43) Date of publication of application: **25.09.2002**
(21) Application number: 01201107.8
(22) Date of filing: 23.03.2001
(51) Int. Cl.: G01N 33/74, G01N 33/574, G01N 33/533

(54) **Short turnaround time insulin assay**

(71) Applicant: Future Diagnostics B.V., 6603 BT Wychen (NL)
(72) Inventor: Martens, Michael F. W. C., 5704 HC Helmond (NL); Rosmalen, Franciscus M.A., 6602 EP Wychen (NL)
(74) Representative: Prins, Adrianus Willem

(57) **Abstract**

The present invention relates to a real-time test system for use in methods to analyse samples on insulin. Particularly, the invention makes it possible to carry out the analysis close to a site wherein the test results are needed. The present invention is especially suitable in intra-operative applications. The invention can be used for *in* and *ex vivo* applications.

## Description

The present invention is in the field of insulin-related or -associated diseases and discomforts. More in particular, it relates to a fast insulin assay with a short turnaround time. The invention makes it possible to carry out the assay or analysis close to a site wherein the test results are needed. The present invention is especially suitable in intra-operative applications. The invention can be used for *in* and *ex vivo* applications.

Insulin is produced by beta cells in pancreatic islets of Langerhans via a complex process of proteolytic conversion. The precursor pro-insulin is transported to the Golgi apparatus where it is packed into secretory granules. Maturation of the secretory granules is associated with conversion of pro-insulin to insulin and C-peptide by enzymatic cleavage. Secretion of insulin into the bloodstream is accompanied by the release of small amounts of proinsulins.

There are quite a number of diseases and discomforts associated with and related to abnormal insulin excretions. The present invention is useful in this field.

For instance, with an incidence of 4 cases per million persons per year, insulinomas are the most common islet cell tumors. In 80% to 90% of these cases, organic hypoglycemia and hyperinsulinism are secondary to a sporadic, solitary benign insulin-secreting intrapancreatic tumor. Preoperatively, tumors with a size of about 1 cm and higher can be detected using a number of techniques. Among available techniques for pancreatic imaging, morphologic tools such as somatostatin receptor scintigraphy, endoscopic ultrasonography (USG), helical computed tomography (CT), or functional localization with intra-arterial stimulation (IAS) tests appear to be of particular value.

In Surgery, 128 (2000) 386-391, Berends *et al.* describe that the various known pre-operative imaging techniques to find insulinomas have a success rate of about 60%. In addition, they describe that laparascopic detection by laparoscopic ultrasonography could identify insulinomas in 90% of the patients.

Hashimoto and Walsh describe in an article in Elsevier Science Inc., 189 (1999) 368-373 that preoperative localization of insulinomas is not necessary. On the basis of statistics, they conclude that the diagnosis of an insulinoma does not require extensive localization studies before operation. More in particular, they come to this conclusion because the combination of surgical exploration and intraoperative ultrasonography identified more than 90% of insulinomas. The resections and enucleations are retrospectively reviewed.

Intraoperatively, a careful pancreatic manual exploration completed with intraoperative ultrasonography (IOUS) leads to the detection of about 90% of these solitary insulinomas. Virtually all patients with such a solitary lesion are eventually cured after the first surgical exploration by enucleation of the tumor(s) when feasible or formal pancreatic resection when necessary. Conversely, surgical treatment of organic hypoglycemia may prove much more demanding in patients with specific pathologic conditions such as multiple insulinomas in the setting of multiple endocrine neoplasia type I (MEN-1), metastatic insulin-secreting carcinoma, or nesidioblastosis. The disease is then often multifocal or diffuse, and preoperative localization is rarely exhaustive. For these patients, a fine balance must be struck between removing total gross tumor and limiting prophylactic resection to prevent endocrine and exocrine pancreatic insufficiency.

For the last two decades, various insulin measurement techniques have been used for the management of insulinomas. Functional localization of insulinomas by measurement of insulin gradients after percutaneous venous sampling of the portal venous system was first described in 1975 by Ingemansson *et al.* in Surg. Gynecol. Obstet. 141 (1975), 705. Doppman *et al.* (Radiology 178, (1991), 237) further improved the potential of the functional localization of insulinomas by coupling intra-arterial calcium injection and insulin measurement in the suprahepatic veins (selective arterial calcium injection test).

Like most other endocrine peptides, insulin has a short half-life in the systemic circulation. Serum insulin levels decrease rapidly after resection of the oversecreting tissue. With the development of techniques for extemporaneous hormonal assay, intraoperative hormone measurements have been proposed to confirm the completeness of the surgical resection in various endocrine diseases. Rapid radioimmunologic insulin assays have been described for almost two decades, but reports of intraoperative insulin measurement during surgical removal in insulinoma remain anecdotal. In fact, in the medical practice insulin measurements are only retrospectively used.

In this light, it is noted that known insulin immunoassays, which are used in practice, consist of radioimmunoassays using polyclonal antisera which cross-react with proinsulins, and two-site assays using monoclonal antibodies. These immunometric assays have led to improvements in specificity and sensitivity as compared to radioimmunoassays. However, these known and practically assays require specific laboratories, where the tests are carried out, and which, hence, require transportation of the samples to be tested and available measuring time and planning before test data are generated. The involvement of such specific clinical or chemical laboratories lead to the situation that the time between sampling and availability of the results in practice is more than 1 day.

The main aim of the present invention is, however, the provision of an insulin measurement that can be used prospectively, and hence intraoperatively.

In the World Journal of Surgery, 22 (1998) 1218-1224, Proye *et al.* describe that they have measured insulin intraoperatively during surgical management of insulinomas. Particularly, they describe that they have used intraoperative insulin measurements to guide the surgical treatment of various diseases, such as sporadic insulinomas, multiple endocrine neoplasia, insulin secreting carcinoma and pancreatic nesidioblastosis, diseases for which also the subject of the present invention is useful. More in detail, insulin was measured with a radioimmunologic assay in blood samples simultaneously drawn from a peripheral vein and the portal vein at the beginning of the operation and 20 minutes after the tumor removal. At the beginning of the procedure, after exposure of the portal vein and before any tissue was resected, blood samples were drawn simultaneously from the portal vein and a peripheral vein. Then, standardized surgical explorations were performed, including exhaustive visual inspection and palpation from the inframesocolic area to the supramesocolic area. A Kocher's maneuver was performed and the posterior aspect of the pancreas tail was dissected. The whole pancreatic gland was subsequently manually palpated and further examined with intraoperative ultrasonography. Any detectable pancreatic tumor was then resected by enucleation or formal pancreatic resection. Twenty minutes after resection, simultaneous systemic and portal blood samples were drawn again.

The blood samples were tested on the serum levels of insulin using a radioimmunologic assay using polyclonal anti-insulin or anti-C peptide serum (Riagnost Insulin *ex* Hoechst-Behring) or a more specific immunoradiometric assay using monoclonal antibodies for insulin (Bi-Insuline IRMA *ex* Diagnostics Pasteur). The results of the tests were available within about 60 minutes.

By the nature of the test, this method requires that during the operation radiometric apparatuses outside the operating room need to be stand-by. Further, for an intraoperative assay 60 minutes is quite a long time, giving infection risks and so on. The present invention aims to solve these two problems in that it aims to provide a method that can be carried out in the operating room and that does not take that long.

Moreover, it would be highly desirable to have a method that makes it possible to determine or locate any excess insulin producing parts of the pancreas in a quantitative way, since that makes it possible to provide an alternative for or additional method to locate for instance insulinomas.

The above-identified aims are reached and the sketched problems are solved by the present invention. The present invention provides for a system that can be carried out on the spot. In addition, it provides a test method using equipment that can be moved or transported to each suitable room without difficulties. It relates to a test procedure, wherein test results become available within about 20 minutes, i.e. it is possible to have the test results available within 20 minutes after blood samping; the test is real-time, reliable and provides the possibility of quantitative assessment of insulin, which makes the test really suitable for intraoperative applications. For example, it becomes possible to prospectively determine in which part of the pancreas an insulinoma, irrespective of its size, is located, and to determine intraoperatively whether the insulinoma(s) are removed.

More in detail, the present invention relates to a real-time test system comprising at least one reservoir with monoclonal anti-insulin or anti-C peptide capture antibodies solidified in said reservoir, which reservoir is capable to receive a sample; a wash solution; labelled monoclonal anti-insulin or anti-C peptide antibodies useful as a tracer, wherein the label allows photometrical detection; and at least one photomultiplier detector. In a preferred embodiment, in the test system according to the invention the labelled monoclonal anti-insulin or anti-C peptide is present in dried form in the said reservoir. In the system of the invention the reservoir preferably is a microtiter well. Moreover, since generally also standards and controls are used, the system of the invention is in the form of a microtiter well-plate.

In a preferred embodiment, the said labelled monoclonal anti-insulin or anti-C peptide antibodies are labelled by a chemiluminescent label.

In a further aspect, the invention relates to a method for determining insulin levels in a sample, comprising adding the sample to a reservoir with monoclonal anti-insulin or anti-C peptide capture antibodies solidified in said reservoir, and labelled monoclonal anti-insulin or anti-C peptide antibodies useful as a tracer, followed by incubation giving labelled insulin complexes; washing; and detecting the labelled insulin complexes photometrically.

The insulin assay or test system of the invention allows for a direct and fast quantitative determination of the insulin hormone. This fast test is real-time; it allows to give results in less than 30 minutes, and preferably even in less than 12 minutes. The system only contains elements that make it possible that the test is carried out at the site where the test results are needed. The test system is hence in principle easily transportable.

This assay utilizes two monoclonal antibodies against different epitopes on the insulin molecule or of the C-peptide. Such monoclonal antibodies are commercially available and/or can be prepared using standard techniques: e.g. the C- and N-parts of insulin are separately used to immunisate mice; after sacrificing the mice spleen cells are fused with non-producing myeloma cells, followed by conventional selection, re-cloning to clonal, purification and concentration steps. One type of monoclonal anti-insulin or anti-C peptide antibody is attached to a solid surface in the reservoir, e.g. coated onto the surface of the reservoir, which preferably is a microtiter well. The other monoclonal antibody directed against another part of the insulin molecule is labelled with a label that allows photometrical detection. The insulin assay of the present invention preferably is a one step, two-site chemiluminescent immunometric assay. Suitable systems are based on the well-known accusphere technology of Organon Teknika. In a preferred embodiment the second antibody is labelled with isoluminol. The labelled second antibody, preferably is present in a dry form in the reservoir. More in particular, the test system of the present invention makes use of a microtiter strip plate that is ready to use.

In use, generally, first a standard curve is made. To check the validity of this standard curve controls are measured. When the controls are within the indicated area, the system is ready to receive and measure samples such as serum plasma, EDTA plasma, perfusion plasma or all culture supernatant. The insuline containing samples are introduced in the antibody containing reservoir. Preferably, this is done by means of a multi-channel pipette. The sample, standards and controls are incubated for a short predescribed time, in the preferred embodiment 7 minutes at room temperature. The incubation is generally carried out while shaking the reservoirs, for instance on a STAT-Shake (*ex* Future Diagnostics B.V., Wijchen, The Netherlands). During this incubation step sandwich complexes are formed. Unbound-labelled antibody is removed by a wash step, for instance with a PBS wash buffer, while using a STAT-Wash (*ex* Future Diagnostics B.V., Wijchen, The Netherlands). The washing step is followed by the detection protocol. When the label is a chemiluminescent label, this generally encompasses the use of activator solutions. In a preferred embodiment, the label is luminol and the activation makes use of a sodium hydroxide solution (4%), and a peroxide solution (0.12%). For this activation step suitable use can be made of a STAT-Read (*ex* Future Diagnostics B.V., Wijchen, The Netherlands) which automatically injects two activator solutions, initiating the chemiluminescence reaction. The amount of label is detected using a photometrical method, generally involving a photomultiplier. The time between obtaining a blood sample and reading a result can be as low as about 10 minutes.

This fast, preferably one step insulin assay offers a quick, reliable, and quantitative result of insulin levels and may be used as an intra-operative tool in the localization of, for instance, insulinoma tumors in the pancreas and monitoring the effectiveness of surgical treatment of insulinomas.

By way of example, the present invention is now described in detail for the localization and removal of insulomas. In this light, it is noted that the pancreas body and tail have a limited number of connections to the *Vena splenica,* whereas the neck and head of the pancreas additionally also have a limited number of connections to the *Vena porta.* An insulinoma is a tumor that hypersecretes insulin sometimes up to 10,000 times the normal production. By determining the insulin content of blood samples taken at different spots in the *Vena splenica* and *Vena porta,* the medical professional can within 20-30 minutes dependent on the influx of insulin in the *Vena splenica* and *Vena porta* easily and accurately determine where an insulin secreting tumor is situated in the pancreas. Moreover, the invention makes it possible that after the enucleation or resection, the surgeon can on the basis of an additional sampling in one of or both the *Vena splenica* and *Vena porta* within 20-30 minutes determine whether the operation can be completed or that there are still hyperexcreting tumor cells left.

The samples can, e.g. and by preference, be taken while using a probe that can be brought in the *Vena splenica* and *Vena porta* for instance from the groin, and that is arranged in such a way that blood samples can be collected.

In a particular aspect, the present invention, hence, also relates to a method for determining insulin levels, comprising sampling blood in the *Vena splenica* and/or *Vena porta*, comprising the steps of introducing a probe in one of said veins, sampling at one or more spots in the said vein(s), and analysing the samples using the above-described general method of the invention.

In a preferred embodiment, the present invention relates to a system comprising a probe arranged to take samples from veins in the body; and the test system of the invention. In particular, this system comprises a probe arranged to be introduced in the *Vena splenica* and/or *Vena porta.*

The subject of the present invention can also be used as a quick, real-time insulin test when a decision is to be made on whether a particular pancreas can be used for transplantation.

At present, in case of a transplantation a pancreas available for transplantation must be brought in a patient's body within 6 hours after removal from the donor. In these 6 hours, the pancreas must often be transported, and subjected to a number of tests. One of the tests encompasses culturing of pancreas cells after washing, followed by measurement of the insulin production. The test is carried out in specialized laboratories, and on average the test results are available within about 24 hours. At that time, the pancreas is already placed in the patient's body, which makes that if the test gives negative or unfavourable results in a worst case scenario a second operation may be needed to remove the transplanted pancreas.

The assay and techniques of the present invention make it possible to have the test data available on the spot and within less than 20 minutes, preferably in about 10 minutes. The test data can hence be used to take the decision whether or not the transplantation should be carried out.

Until the moment of the present invention, wide-scale use of insulin assays was a subject of research rather than a diagnostic application. Spontaneous hypoglycaemia, a disorder which can be caused by hyperinsulinism, insulinoma, insulin autoimmune syndrome and non-insulinmediated factors, was almost the only clinical indication for the measurement of plasma insulin. Diabetes mellitis is diagnosed solely on the basis of chronic hyperglycaemia. Thus, measurement of plasma insulin has no clininal value in the diagnosis or management of diabetic patients, with the exception of rare cases, including the syndrome of severe insulin resistance and abnormalities in beta-cell secretory products. Otherwise, insulin measurement is used in experimental investigations to study the pathophysiology of various disorders, especially diabetes.

However, with the provision of the present invention such insulin tests become practically usable in diagnosis. For instance, the assay and method of the invention is of practical use is in the provision of individual protocols for patients suffering from diabetes mellitis. More in particular, the quick test of the invention makes it possible that for patients for which diabetes mellitis was diagnosed, the need of insulin can be determined on the basis of the reaction of the body to the intake of carbohydrate and in particular sugar containing food. This reaction of the body, of course, is patient dependent. To determine reference values and limits, insulinaemia must be measured in normoglycaemic subjects with a normal body weight. Moreover, as insulinaemia is most often measured during stimulation tests, reference values must also be determined for the most common tests such as the oral glucose tolerance test or the intravenous glucose tolerance test.

The present invention makes it possible to have for each individual patient a very specific protocol for stimulation tests can be carried out within 1 or 2 days to determine the optimal insulin requirements. Thereto, blood samples taken before and after the uptake of a particular food are analysed for insulin using the assay of the invention. Moreover, the present invention allows that a medical professional is no longer required. The assays can be carried out by an assistant.

In a further aspect, the present invention is practical in use in a method wherein beta cells, generally of human or porcine origin, are cultured *in vitro* or *ex vivo.* The beta cells are monocellular and should be well-producing in respect of insulin. The aim of these cultured beta cells is to be implanted in the pancreas of a patient suffering from hypoinsulaemia. The present invention allows to check right before the implantation whether the cultured beta cells after transportation to the medical professional who carries out the implantation still have the required insulin producing activity. The medical professional can determine at the spot whether suitable beta cells are obtained. Moreover, the activity of the beta cells after implantation can be checked by the present invention as well.

The present invention will now be further illustrated while referring to the following, non-limiting examples.

### Example 1 (Best Mode)

Components in the preferred insulin assay kit of the invention:
1. Ready To Use Strip Plate (P1):
   A microtiter strip plate (32 wells) is coated with monoclonal anti-insulin or anti-C peptide as capture antibody. An isoluminol labelled monoclonal anti-insulin or anti-C peptide antibody is present in the well as a tracer antibody in dried form.
2. Insulin Zero Standard (SO):
   1 vial labelled 'SO' contains lyophilized insulin-free human serum albumin.
3. Insulin Standards (S1, S2, S3, S4, S5):
   Vials labeled 'S1" through 'S5' contain a standard amount insulin in an insulin-free human serum albumin in lyophilized form. The standards are calibrated against the 1^{st} WHO International Reference Preparation (IRP) of Insulin (code 66/304).
4. Insulin Controls (C1 and C2)
   Vials labeled 'C1' (Control 1) and 'C2" (Control 2) contain a controlled amount of insulin in an insulin-free human serum albumin in lyophilized form.
5. PBS Wash buffer (10 x concentrated) (W 1)
   One vial labeled 'W1' contains 30 mL of 10 times concentrated phosphate buffered saline with 0.09% sodium azide as a preservative.
6. Non-coated Strip Plate (P2):
   A microtiter strip plate (32 wells), with natural colored non-coated wells for the purpose of collecting and aliquoting of standards, controls and patient plasma samples.
7. Activators (A1 and A2):
   Two bottles labeled 'A1' (Activator 1) and 'A2' (Activator 2) each contain 20 mL Activator Solutions to generate the chemiluminescence light signal. Activator 1 (A1) contains 4% sodium hydroxide (NaOH). Activator 2 (A2) contains 0.12% peroxide.

### Additional materials and instruments required

1. Precision pipettes: 150 µl and 1000 µl
2. Distilled or deionized water
3. Timer
4. EDTA collection tube
5. Serum collection tube
6. Future Diagnostics STAT-Centrifuge; STAT-Shake; STAT-Wash; and STAT-Read
7. Measuring glass of 300 ml

### Reagent preparation and storage

1. The Ready to Use Strip Plate (P1) should be at room temperature;
2. Insulin Zero Standard (SO) is reconstituted in the vial labelled 'SO' with 2.0 ml of distilled or deionized water. The vial is allowed to stand 15 minutes at room temperature, then mixed by gentle shaking to ensure complete reconstitution;
3. Insulin Standards (S1 - S5) are reconstituted in the vials labelled 'S1' through 'S5' with 1.0 ml of distilled or deionized water. The vials are allowed to stand 15 minutes at room temperature, then mixed by gentle shaking to ensure complete reconstitution;
4. Insulin Controls (C1 and C2) are reconstituted in the vials labelled 'C1' and 'C2' with 1.0 ml of distilled or deionized water. The vials are allowed to stand for 15 minutes at room temperature, then mixed thoroughly by gentle shaking to ensure complete reconstitution;
5. Wash Solution (W1) is diluted with 270 ml of distilled or deionized water and mixed;
6. Non-coated Strip Plate (P2) is sampled;
7. Activator Solutions (A1 and A2) should be stored at 15-30°C until expiration date on vial;
The determination of Insulin should be performed in serum, EDTA plasma, in perfusion solution or in cell-culture supernatent. It is recommended to perform the assay in duplicate, for this purpose at least 300 µl of patient sample perfusion solution or cell-culture supernatant is needed.
1. Proper sample collection from the patient requires one, non-diluted blood sample to be drawn in glass tubes with EDTA as anticoagulant (i.e. Lavender top Vacutainer, Becton Dickenson or equivalent) and is shaken head over tail for 5 times to ensure proper anticoagulant mixing. Alternative for serum the blood should be collected in a Vacutainer (no additives). Separate either plasma or serum from cells as soon as possible, by means of centrifugation (10 min. at 2000 g-force).
2. If the plasma or serum is analyzed at a later date the plasma or serum sample should be frozen immediately.
3. Just prior to setting up the insulin assay the frozen patient samples must be thawed.

### The insulin test procedure

### Procedure

- Label the micro titer strips, 1 to 4, of the non-coated strip plate 'P2' and pipette in duplicate in vertical sequence of the wells (A1-H1)(A2-H2) etc., 150 µl of: Zero Standard (SO), Standards (S1-S5), Controls 1 and 2 (C1, C2), and patient plasma sample.
- Label the microtiter strips, 1 to 4, of the ready to use strip plate 'P1", identical to the labelling of the non-coated strip plate 'P2'.
- Transfer with the a 100 µl pipet the appropriate Standards, Controls and patient samples from the non-coated strip plate 'P2', directly into the bottom of the wells of the ready to use strip plate 'P1'.
- Place the ready to use strip plate, 'P1' on the STAT-shake immediately and incubate for exactly 7 minutes at room temperature.
- Wash the strips in the STAT-Wash using 300 µl of diluted wash solution. Repeat 2 times.
- The washed strips are measured in the STAT-Read within 15 minutes after washing of the strips.
- Count each micro titer strip for 3 seconds in the STAT-Read using Activator A1 and Activator A2 respectively, 100 µl each. The entire chemiluminescence reaction is complete in 3 seconds. This reaction is irreversible, so each well can only be read once.
- Patient samples with values greater than the highest standard (S5) may be diluted with Zero Standard (SO) and re-assayed. Result values must be multiplied by the dilution factor.

The insulin assay of the invention allows for an analytical sensitivity of 4 pmol/L and a dynamic range up to 2000 pmol/L. The intra-assay variation at 65, 359 and 1029 pmol/L are 5.2%, 8.2% and 6.7% respectively. Inter-assay variation at 37, 212 and 632 pmol/L are 9.1%, 9.3% and 10.7% respectively. The mean per sample of high-low recovery and parallelism are within 83-103%. The cross reactivity with Glucagon, C-peptide and Pro-Insulin was < 0.5% and no high dose hook was observed at concentrations > 200,000 pmol/L.

The Insulin assay was compared to a commercially available Insulin Enzyme Immuno Assay (EIA), on a population of 41 samples. The range of values obtained using the commercially available insulin EIA (A) ranged from 11.2 to 751 pmol/L. Values obtained with the Insulin Assay (B) ranges from 16.5 to 1232 pmol/L. A correlation coefficient of (r) = 0.99 with a regression formula Y = 1.72X - 4.2 was obtained from linear regression analysis of the data.

### Example 2

In 1994, a patient suffered from collapses appearing temporarily during the day and losses of consciousness that manifested as a loss of sight and speaking ability lasting for seconds. He underwent a blind insulinoma resection.

In order to find the tumor which caused the hypersecretion of insulin several ultrasonographs (USG) and CT scans have been performed. No focal changes have been found. The decision to perform the operation without localization was made.

No focal changes were found during the operation. The decision to perform "blind" resection of the body and tail of the pancreas was made. Although the symptoms were less severe, the patient was not cured. Five months after the operation the patient started to take diazoxyde at a dose of 2 x 100 mg. This dose went up to 600 mg per day.

In 2000, that patient suffered from general malaise appearing temporarily and losses of consciousness lasting for seconds and appearing 2-3 times a month. Upon testing, he showed elevated insulin levels.

To locate the tumor, USG, CT scan and ASVS (Arterial Stimulating and Venous Samping) was performed. The results of all these examinations were negative. The decision of re-operation was made.

At the beginning of the operation, a blood sample was taken from a peripheral vein and the insulin level was measured with the insulin assay of of example 1. The result was 84 pmol/l. Then, the pancreatic gland was examined very carefully after having done the Kocher's maneuver. Neither visible nor palpable focal changes were found. More insulin levels were measured intraoperatively using the assay of the invention. Blood samples were taken from the mesenteric superior vein at the lower edge of the pancreas, the mesenteric superior vein in the middle of the gland, the portal vein and splenic vein. The results were: 832 pmol/1, 463 pmol/l, 302 pmol/l and 106 pmol/l insulin, respectively. Intraoperative ultrasound examination confirmed the presence of occult tumor located in the middle part of the head of pancreas. The tumor was removed, then the insulin levels from the places mentioned above were measured again - to confirm that the removed tissue was responsible for the hypersecretion of insulin. The results were 68 pmol/l, 70 pmol/l, 73 pmol/l and 103 pmol/l, respectively. Intraoperative pathological examination revealed that the tumor was an *insulinoma.* Considering the fact that the post-excision insulin levels were much lower than the pre-excision insulin levels, the presence of another tumor was rather unlikely. The decision to end the operation was made.
After the procedure, temporary hyperglycemia occurred, but the infusions of insulin were not necessary. Normoglycemia was achieved within 3 days. In the postoperative course, pancreatic fistula and then subphrenical abscess appeared. Both these complications were treated operatively. Glucose levels were within normal limits and no symptoms of hypoglycemia were observed.

### Example 3

As indicated in the general description (vide supra), now a surgeon's judgement of a successful pancreas transplantation is the return of a typical pink color of the transplanted pancreas and the ability of the transplanted pancreas to lower the glucose level. While using the assay of the present invention, prior to the transplantation, the well-functioning of the pancreas can be tested by measuring the insulin-level in the perfusion solution upon stimulating the pancreas with increasing concentrations of glucose. This is hard to quantify because each pancreas would react differently to the added dose of glucose. However, as a response to the stepwise addition of glucose the well-functioning pancreas should show a comparable stepwise increase in insulin secretion into the perfusion solution.

After the successful transplantation the surgeon can measure peripherally the concentration of insulin (or C-peptide for that matter), which should preferably be within normal range (8-20 mIU/liter) dependent on the level of glucose in the patients blood system.

### Example 4

With the assay of the invention, prior to implantation of cultured beta cells, the supernatant of the culture can be measured at the spot for insulin levels (or C-peptide levels) upon treating the cell culture with glucose. The level of secreted insulin depends on the concentration of beta cells in the culture. Properly producing beta cells should show a dose-dependent secretion.

After the successful implantation the surgeon can measure peripherally the concentration of insulin (C-peptide), which should be preferably within normal range (8-20 mIU/liter), dependent on the level of glucose in the patients blood system.

### Example 5

With diabetes patients the glucose tolerance can be tested by measuring the immediate response (and in time) of the insulin levels, while using the assay of the invention.

### Example 6

Also real-time measurements upon the intake of oral beta-cell stimulating drugs can be carried out to fine-tune the concentration of the drugs, at the spot while using the assay of the invention.

## Claims

1. Real-time test system comprising at least one reservoir with monoclonal anti-insulin or anti-C peptide capture antibodies solidified in said reservoir, which reservoir is capable to receive a sample; a wash solution; labelled monoclonal anti-insulin or anti-C peptide antibodies useful as a tracer, wherein the label allows photometrical detection; and at least one photomultiplier detector.

2. Test system according to claim 1, wherein the labelled monoclonal anti-insulin or anti-C peptide is present in dried form in the said reservoir.

3. Test system according to claim 1 or 2, wherein the said labelled monoclonal anti-insulin or anti-C peptide antibodies are labelled by a chemiluminescent label.

4. The system of any one of the preceding claims, wherein the reservoir is a microtiter well.

5. A method for determining insulin levels in a sample, comprising adding the sample to a reservoir with monoclonal anti-insulin or anti-C peptide capture antibodies solidified in said reservoir, and labelled monoclonal anti-insulin or anti-C peptide antibodies useful as a tracer, followed by incubation giving labelled insulin complexes; washing; and detecting the labelled insulin complexes photometrically.

6. The method of claim 5, wherein the sample is perfusion solution obtained from a pancreas removed from the body after stimulation said pancreas with an insulin-production influencing compound, preferably glucose.

7. The method of claim 5, wherein the sample is supernatant of *in vitro* cultured beta cells.

8. The method of claim 5, wherein the sample is a blood sample.

9. A method for determining insulin levels, comprising sampling blood in the *Vena splenica* and/or *Vena porta,* comprising the steps of introducing a probe in one of said veins, sampling at one or more spots in the said vein, and analysing the samples using the method of claim 5.

10. System for carrying out the method of claim 6 comprising a probe arranged to be introduced in the *Vena splenica* and/or *Vena porta* and the test system of any one of claims 1-4.
